Europäisches Patentamt

European Patent Office

Office européen des brevets

(11) Publication number: **0 390 085**
**A2**

# (12) EUROPEAN PATENT APPLICATION

(21) Application number: **90105841.2**

(22) Date of filing: **27.03.90**

(51) Int. Cl.5: **H04N 1/32**

(30) Priority: **27.03.89 JP 75667/89**

(43) Date of publication of application:
**03.10.90 Bulletin 90/40**

(84) Designated Contracting States:
**DE NL**

(71) Applicant: **Kabushiki Kaisha Toshiba**
**72, Horikawa-cho Saiwai-ku**
**Kawasaki-shi(JP)**

(72) Inventor: **Toshimitsu, Akihiro, c/o Intellectual**
**Property Div**
**Kabushiki Kaisha Toshiba, 1-1 shibaura**
**1-Chome, Minato-ku, Tokyo 105(JP)**

(74) Representative: **Blumbach Weser Bergen**
**Kramer Zwirner Hoffmann Patentanwälte**
**Radeckestrasse 43**
**D-8000 München 60(DE)**

(54) **Method and system for controlling transmission of image data.**

(57) In a network interface unit for a medical image data communication storage system, when a packet is received by ACR-NEMA interface, a packet type data of the received packet is compared with a preset packet type data. If the packet types coincide with each other, the received packet is stored in an image memory (43) through an image data bus (45), and is then transferred to a transmitting line (59). If the packet types do not coincide with each other, the received packet is input to a CPU (40) through a control data bus (44), and is then transferred to the transmitting line (59).

F I G. 6

EP 0 390 085 A2

The present invention relates to a method and system for controlling transmission of image data.

International communication protocol standards in a medical image data storage communication system include ACR (American College of Radiology) - NEMA (National Electrical Manufactures Association) standards. Medical image data communication is performed through a network interface unit and a network using an ACR - NEMA communication protocol based on the above standards.

Conventionally, medical image data acquired by a medical image acquiring apparatus has been transmitted to other apparatuses through a network interface unit and a network as packet data based on the ACR - NEMA standards without changing a format. Therefore, it takes a long time to perform data communication, thus degrading transmission efficiency.

Thus, in medical image data communication, a demand has arisen for developing a network interface unit which can improve transmission efficiency.

It is an object of the present invention to provide a method and system for controlling transmission of image data in a network interface unit.

According to one aspect of the present invention, there is provided a method for controlling transmission of image data in a network interface unit, the method comprising the steps of:
setting a desired packet type;
receiving a packet having a packet type and one of control data and image data;
comparing the packet type of the received packet with the desired packet type; and
selecting one of a control data line and an image data line in accordance with a comparison result.

According to another aspect of the present invention, there is provided a system for controlling transmission of image data in a network interface unit, the system comprising:
means for setting a desired packet type;
means for receiving a packet having a packet type and one of control data and image data;
means for comparing the packet type of the received packet with the desired packet type;
a control data line for transferring the control data;
an image data line for transferring the image data; and
means for selecting one of the control data line and the image data line in accordance with a comparison result.

This invention can be more fully understood from the following detailed description when taken in conjunction with the accompanying drawings, in which:

Fig. 1 is a view showing an arrangement of a medical image data storage communication system;

Fig. 2 is a view showing an arrangement of NIUs and a network in ACR - NEMA communication system;

Fig. 3 is a diagram showing a hierarchical structure in an ACR - NEMA communication protocol;

Fig. 4 is a diagram showing a packet format of a transport/network layer;

Fig. 5 is a diagram showing a packet descriptor word in the packet format;

Fig. 6 is a block diagram showing an arrangement of an NIU according to the first embodiment of the present invention;

Fig. 7 is a diagram showing a slot arrangement on a transmitting/receiving line in a time-division multiplex network shown in Fig. 6;

Figs. 8A and 8B are diagrams showing formats of data to be transmitted/received;

Fig. 9 is a flow chart for explaining an operation of the NIU shown in Fig. 6;

Fig. 10 is a block diagram showing an arrangement of an NIU according to the second embodiment of the present invention;

Fig. 11 is a block diagram showing an arrangement of an ACR - NEMA interface in the embodiments of the present invention; and

Fig. 12 is a flow chart for explaining an operation of the NIU having the ACR - NEMA interface shown in Fig. 11.

Embodiments of the present invention will be described hereinafter with reference to the accompanying drawings.

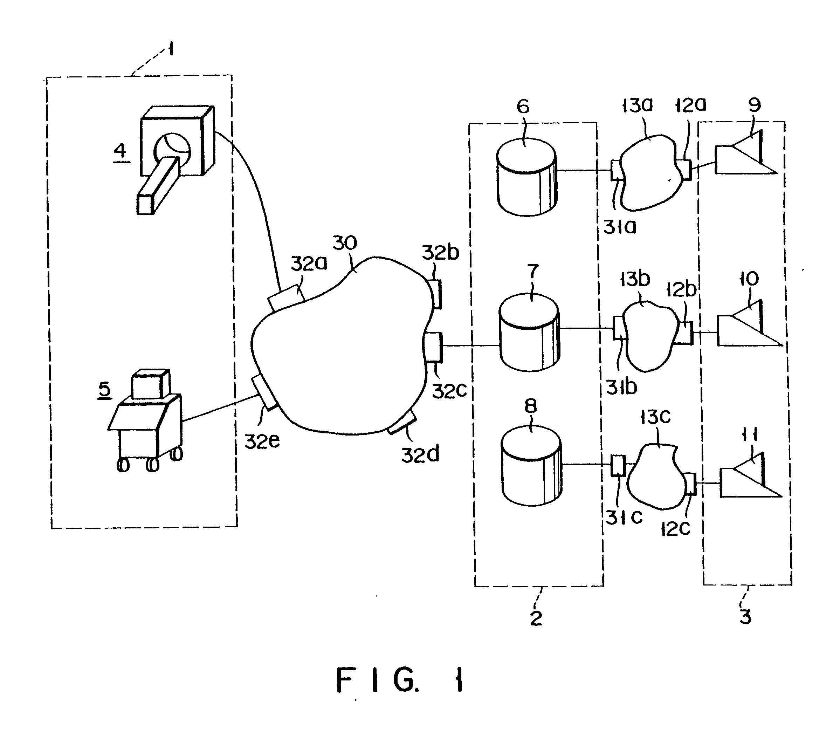

As shown in Fig. 1, a medical image data storage communication system includes a medical image acquiring apparatus 1, a data base 2, a work station 3, network interface units (NIUs) 12a, 12b, 12c, 31a, 31b, 31c, 32a, 32b, 32c, 32d, and 32e, and networks 13a, 13b, 13c, and 30.

The medical image acquiring apparatus 1 includes, e.g., an X-ray CT (Computed Tomography) apparatus 4 and an ultrasonic diagnosis apparatus 5.

The data base 2 includes, e.g., an internal, surgical, and obsterical and gynecological image storage apparatuses 6, 7, and 8.

The work station 3 includes diagnosis apparatuses 9, 10, and 11 for referring to medical images.

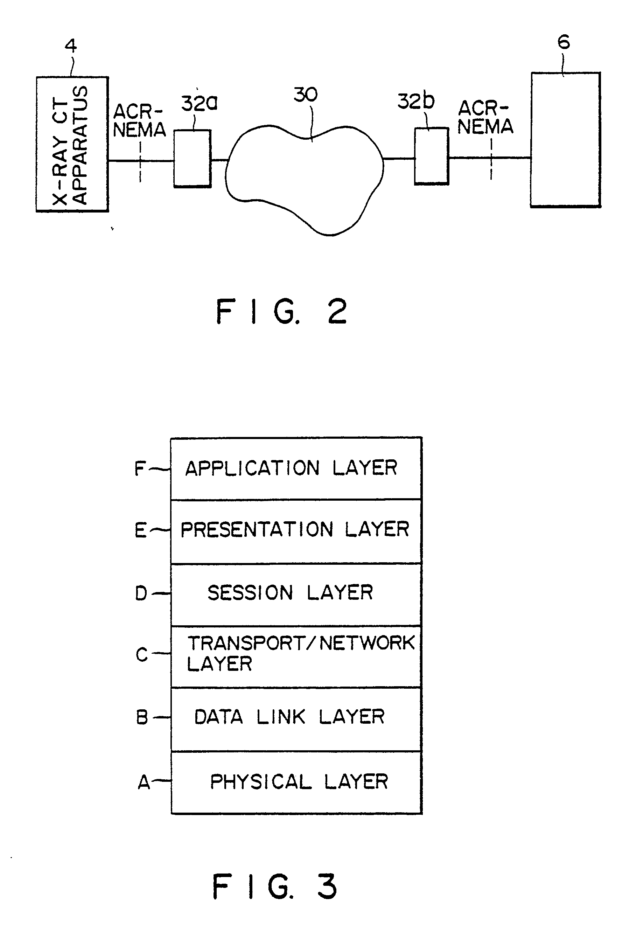

As described above, international communication protocol standards in a medical image data storage communication system include ACR - NEMA standards. Using an ACR - NEMA communication protocol based on the ACR - NEMA standards, data communication between, e.g., the X-ray CT apparatus 4 and the internal image storage apparatus 6 is performed through the NIUs 32a and 32b, and the network 30, as shown in Fig. 2.

The ACR - NEMA standards are associated with a communication protocol having a hierarchical structure. As shown in Fig. 3, this hierarchical structure includes a physical layer A, a data link layer B, a transport/network layer C, a session layer D, a presentation layer E, and an application layer F, in the order named from the lowermost layer.

Data communication using the ACR - NEMA communication protocol is performed in accordance with the following procedures.

When message data (image data and additional data) is transmitted in the application layer F, the presentation layer E converts a format of the message data into a format based on the ACR - NEMA standards.

The session layer D achieves connection with a data communication destination (establishment of connection). The session layer D also cancels the connection with the data communication destination (release of connection) after the message data is transmitted.

The transport/network layer C packetizes and transmits data to the data communication destination using a frame communication function in the layer below the data link layer B.

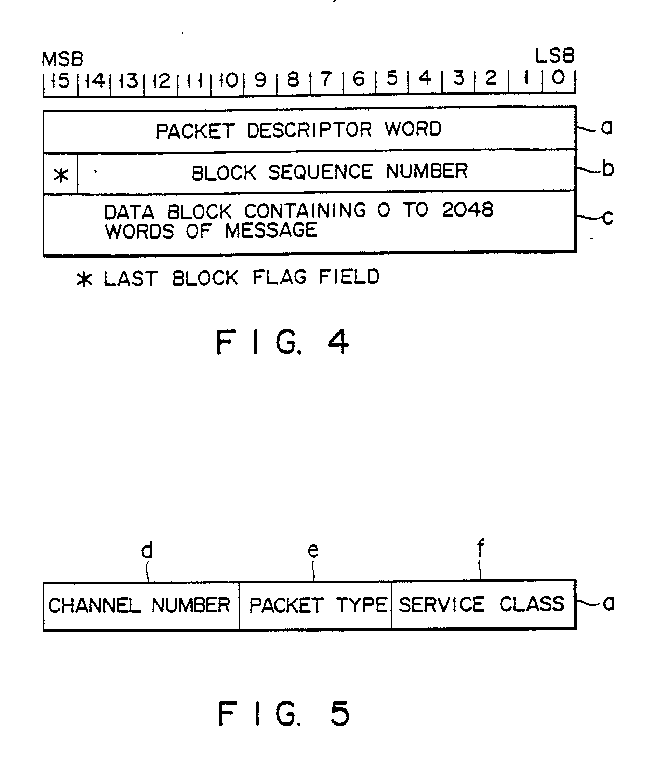

As shown in Fig. 4, a packet format in the transport/network layer C includes a packet descriptor word a, a block sequence number b, and a data block c. Note that the packet descriptor word a includes a channel number d, a packet type e, and a service class f, as shown in Fig. 5.

The packet type in the transport/network layer C is classified into a channel control packet, a command packet, and a data packet. The channel control packet is used to establish or release connection between the apparatuses. The command packet is used to transfer command data. The data packet is used to transfer data except for the command data.

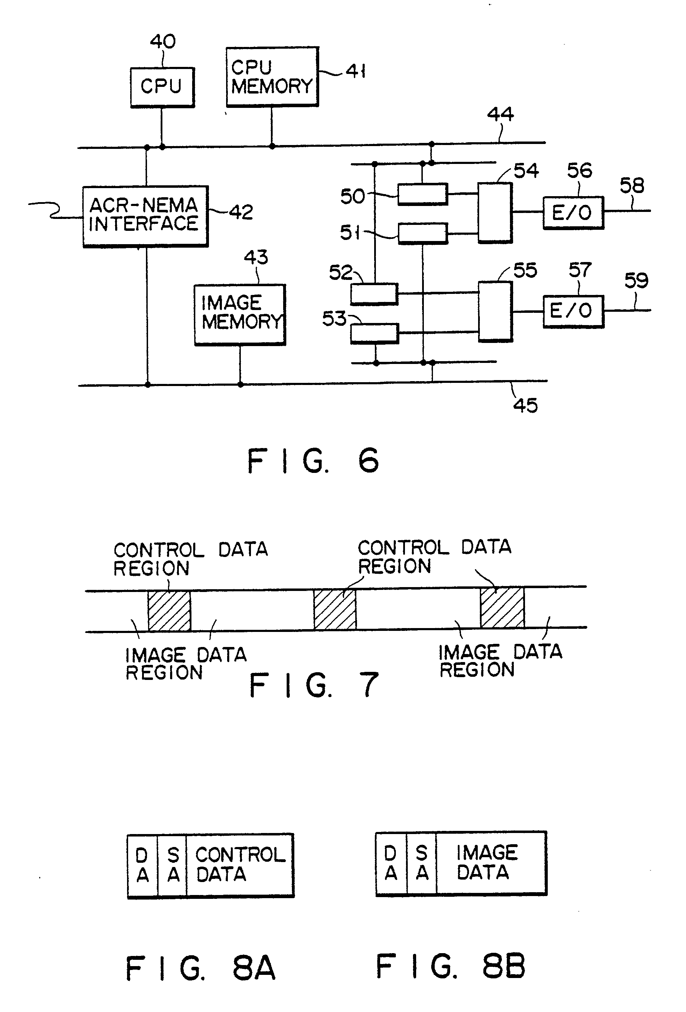

Referring to Fig. 6, the NIU according to the first embodiment is used in time-division multiplex data communication. The NIU includes a CPU (Central Processing Unit) 40, a CPU memory 41, an ACR - NEMA interface 42 for transmitting and receiving an ACR -NEMA packet, an image memory 43 for storing image data, a control data bus 44, an image data bus 45, a receiving control data buffer 50 for storing control data of the received frame, a receiving image data buffer 51 for storing image data of the received frame, a transmitting control data buffer 52 for storing control data of a frame to be transmitted, a transmitting image data buffer 53 for storing image data of a frame to be transmitted, a receiving frame control section 54, a transmitting frame control section 55, E/O (electric/optical) converters 56 and 57, a receiving line 58, and a transmitting line 59. Note that the E/O converters 56 and 57 respectively include O/E converters (not shown). In addition, a plurality of data buffers can be arranged in order to achieve high-speed data processing.

The ACR - NEMA interface 42, the CPU 40, the CPU memory 41, the receiving control data buffer 50, and the transmitting control data buffer 52 are connected to the control data bus 44. In addition, the ACR - NEMA interface 42, the image memory 43, the receiving image data buffer 51, and the transmitting image data buffer 53 are connected to the image data bus 45.

As shown in Fig. 7, the transmitting frame control section 55 transmits control data stored in the transmitting control data buffer 52 and image data stored in the transmitting image data buffer 53 to the transmitting line 59 in accordance with a slot arrangement having control and image data regions, on the basis of formats shown in Figs. 8A and 8B. Note that, in Figs. 8A and 8B, reference symbol DA denotes a destination address; and SA, a source address.

The receiving frame control section 54 receives control data (Fig. 8A) and image data (Fig. 8B) transmitted in accordance with the slot arrangement shown in Fig. 7. When the destination address DA is a self address, the control data and image data are stored in the receiving control data buffer 50 and the receiving image data buffer 51, respectively. The control data stored in the receiving control data buffer 50 is stored in the CPU memory 41. In addition, the image data stored in the receiving image data buffer 51 is stored in the image memory 43.

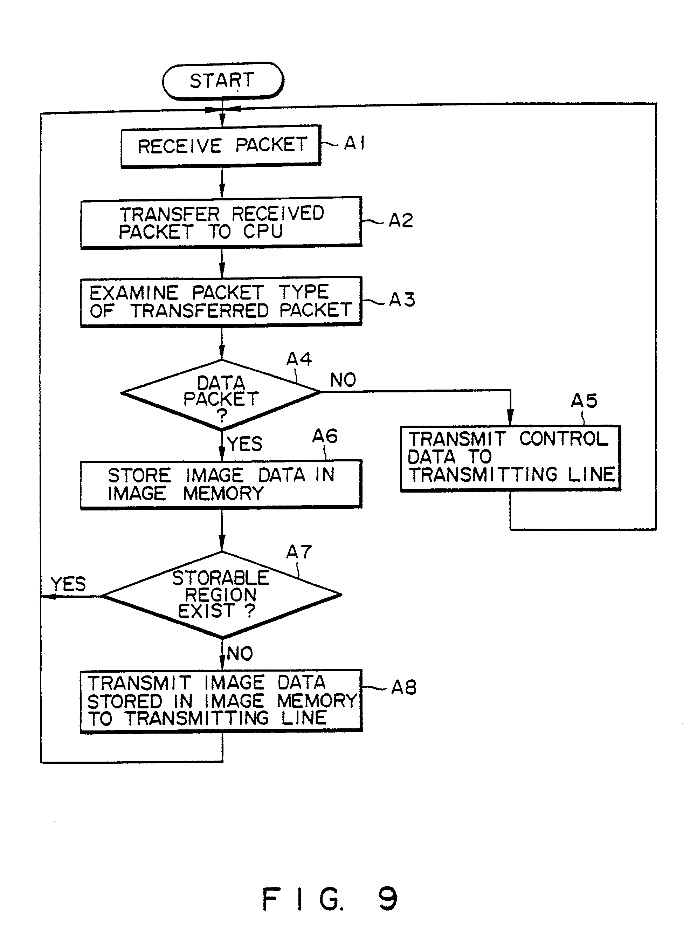

An operation of the NIU according to the first embodiment will be described hereinafter following a flow chart in Fig. 9. Note that a case wherein a packet of the received ACR - NEMA format is transmitted to the network will be described below.

In step A1, a packet of the ACR - NEMA format is received by the ACR - NEMA interface 42. In step A2, the received packet is transferred to the CPU 40 through the control data bus 44. In step A3, a packet type of the received packet is examined by the CPU 40.

If the packet type of the received packet is not a data packet in step A4, the control data in the received packet is stored in the transmitting control data buffer 52. The control data stored in the transmitting control data buffer 52 is transferred to the transmitting line 59 through the transmitting frame control section 55 and the E/O converter 57 (step A5).

On the other hand, if the packet type of the received packet is a data packet in step A4, the image data of the received packet is stored in the image memory 43 (step A6). It is determined in step A7 whether or not a storable region exists in the image memory 43.

If no storable region exists in the image memory 43 in step A7, the image data stored in the image memory 43 is transferred to the transmitting line 59 through the transmitting image data buffer 53, the transmitting frame control section 55, and the E/O converter 57 (step A8).

An NIU according to the second embodiment will be described hereinafter.

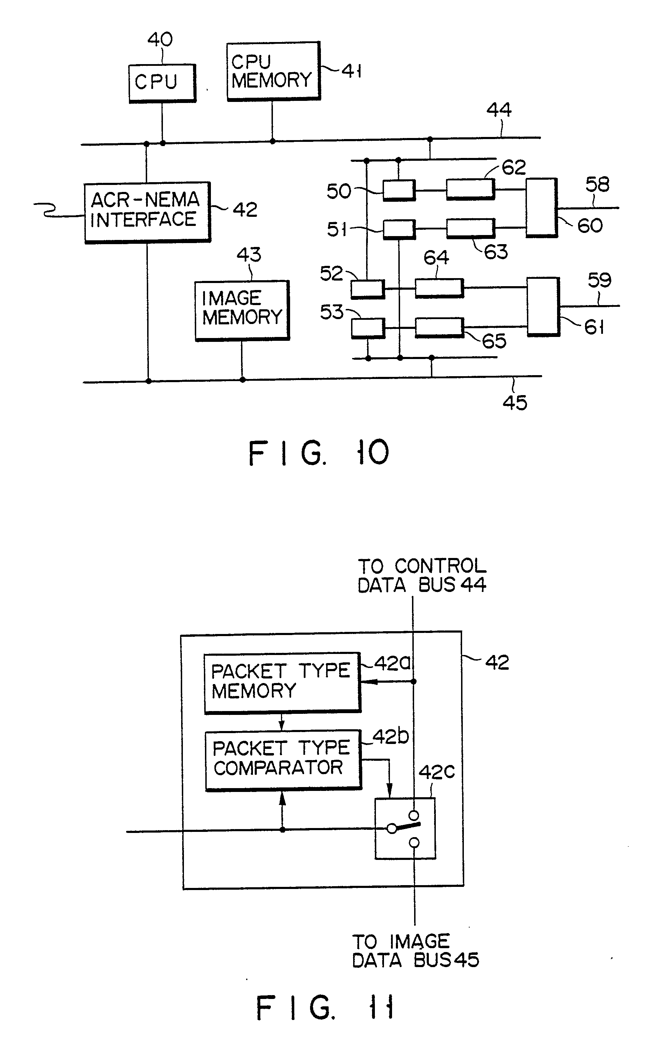

Referring to Fig. 10, the NIU according to the second embodiment is used in wavelength-division multiplex data communication. The NIU includes a CPU 40, a CPU memory 41, an ACR - NEMA interface 42, an image memory 43, a control data bus 44, an image data bus 45, a receiving control data buffer 50, a receiving image data buffer 51, a transmitting control data buffer 52, a transmitting image data buffer 53, a receiving line 58, a transmitting line 59, an optical demultiplexer 60, an optical multiplexer 61, a control data receiving controller 62, an image data receiving controller 63, a control data transmitting controller 64, and an image data transmitting controller 65. Note that the control and image data receiving controllers 62 and 63 have O/E converters (not shown), respectively. In addition, the control and image data transmitting controllers 64 and 65 have E/O converters (not shown), respectively.

The formats of the control and image data are changed into data formats shown in Figs. 8A and 8B by the control and image data transmitting controllers 64 and 65, respectively. The obtained data are E/O-converted, and are input to the optical multiplexer 61. Note that the wavelength of a light beam representing the control data is different from that of a light beam representing the image data.

The optical multiplexer 61 multiplexes light beams respectively representing the E/O-converted image and control data, and the multiplexed beam is transferred to the transmitting line 59.

On the other hand, the optical demultiplexer 60 divides the light beam from the receiving line 58 into light beams respectively representing the control and image data, and the divided beams are output to the control and image data receiving controllers 62 and 63, respectively.

The control and image data receiving controllers 62 and 63 respectively output the control and image data obtained by O/E-converting the input light beams to the receiving control and receiving image data buffers 50 and 51.

The control data stored in the receiving control data buffer 50 is stored in the CPU memory 41. In addition, the image data stored in the receiving image data buffer 51 is stored in the image memory 43.

As described above, since the control data and the image data are distinguished from each other and transmitted in accordance with the packet type of the received packet determined in the CPU 40, transmission efficiency can be improved.

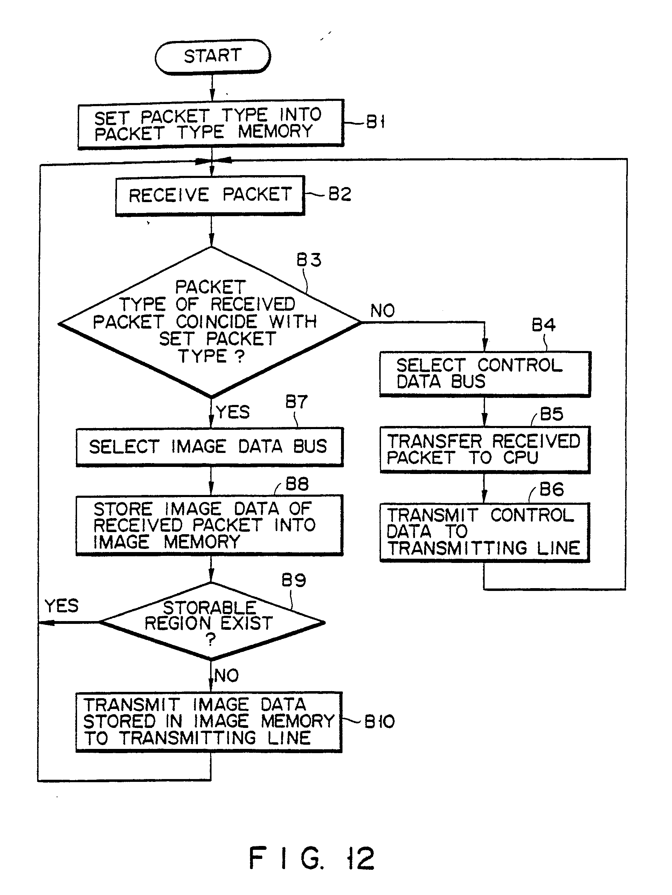

For example, an ACR - NEMA interface shown in Fig. 11 can be used to the embodiment NIU.

In Fig. 11, the ACR - NEMA interface 42 includes a packet type memory 42a, a packet type comparator 42b, and a data bus switch 42c.

The packet type memory 42a stores packet type data representing a packet type designated by the CPU 40 in advance.

The packet type comparator 42b extracts packet type data from the packet descriptor word $\underline{a}$, and compares the extracted packet type data with the packet type data stored in the packet type memory 42a.

The data bus switch 42c selects the control or image data bus 44 or 45 in accordance with the comparison result in the packet type comparator 42b.

An operation of the NIU according to the first embodiment having the ACR - NEMA interface 42 shown in Fig. 11 will be described below following a flow chart in Fig. 12.

In step B1, packet type data representing the packet type designated by the CPU 40 is set in the packet type memory 42a of the ACR - NEMA interface 42.

In step B2, the packet is received.

In step B3, the packet type data of the received packet is compared with the packet type data set in the packet type memory 16a by the packet type comparator 42b.

If the packet types do not coincide with each other in step B3, the control data bus 44 is selected (step B4). Therefore, the data bus switch 42c is switched to select the control data bus 44.

In step B5, the received packet is transferred to the CPU 40. In step B6, the control data of the received packet is transmitted to the transmitting line 59 through the transmitting control data buffer 52, the transmitting frame control section 55, and the E/O converter 57.

If the packet types coincide with each other in step B3, the image data bus 45 is selected (step B7). Therefore, the data bus switch 42c is switched to select the image data bus 45.

In step B8, the image data of the received packet is stored into the image memory 43. It is determined in step B9 whether or not a storable region exists in the image memory 43.

If the storable region does not exist in the image memory 43 in step B9, the image data stored in the image memory 43 is transmitted to the transmitting line 59 through the transmitting image data buffer 53, the transmitting frame control section 55, and the E/O converter 57 in step B10.

As described above, when the packet type of the received packet coincides with the preset packet type, the received packet is not transferred to the CPU 40, but is stored in the image memory 43 through the image data bus 45. After the predetermined image data is stored, the image data is transmitted. Therefore, a processing period required from reception of the packet to the transmission of the data can be shortened. In particular, since the number of packets of medical image data is larger than that of the control data, the processing period can be largely shortened.

## Claims

1. A method for controlling transmission of image data in a network interface unit, characterized by the steps of:
setting a desired packet type;
receiving a packet having a packet type and one of control data and image data;
comparing the packet type of the received packet with the desired packet type; and
selecting one of a control data line and an image data line in accordance with a comparison result.

2. The method according to claim 1, characterized in that the image data line is selected when the packet type of the received packet is a data packet.

3. A system for controlling transmission of image data in a network interface unit, characterized by:
means for receiving a packet having a packet type and one of control data and image data;
means for determining the packet type of the received packet;
first storing means for storing the control data;
second storing means for storing the image data; and
means for transferring the one of the control data and the image data to one of the first and second storing means in accordance with a determination result.

4. The system according to claim 3, characterized in that the image data is stored in the second storing means when the packet type of the received packet is a data packet.

5. A system for controlling transmission of image data in a network interface unit, characterized by:
means for setting a desired packet type;
means for receiving a packet having a packet type and one of control data and image data;
means for comparing the packet type of the received packet with the desired packet type;
a control data line for transferring the control data;
an image data line for transferring the image data; and

means for selecting one of the control data line and the image data line in accordance with a comparison result.

6. The system according to claim 5, characterized in that the image data line is selected when the packet type of the received packet is a data packet.

F I G. 1

EP 0 390 085 A2

F I G. 2

F I G. 3

EP 0 390 085 A2

MSB                                                              LSB
| 15 | 14 | 13 | 12 | 11 | 10 | 9 | 8 | 7 | 6 | 5 | 4 | 3 | 2 | 1 | 0 |

| PACKET DESCRIPTOR WORD | a |
| ✳ BLOCK SEQUENCE NUMBER | b |
| DATA BLOCK CONTAINING 0 TO 2048 WORDS OF MESSAGE | c |

✳ LAST BLOCK FLAG FIELD

# F I G. 4

| CHANNEL NUMBER (d) | PACKET TYPE (e) | SERVICE CLASS (f) | a |

# F I G. 5

F I G. 6

F I G. 7

CONTROL DATA REGION

CONTROL DATA REGION

IMAGE DATA REGION

IMAGE DATA REGION

| D A | S A | CONTROL DATA |
|-----|-----|--------------|

F I G. 8A

| D A | S A | IMAGE DATA |
|-----|-----|------------|

F I G. 8B

F I G. 9

F I G. 10

F I G. 11

START

SET PACKET TYPE INTO
PACKET TYPE MEMORY — B1

RECEIVE PACKET — B2

PACKET
TYPE OF RECEIVED
PACKET COINCIDE WITH
SET PACKET
TYPE ?   B3

NO → SELECT CONTROL
DATA BUS   B4

YES

SELECT IMAGE DATA BUS   B7

STORE IMAGE DATA OF
RECEIVED PACKET INTO
IMAGE MEMORY   B8

TRANSFER RECEIVED
PACKET TO CPU   B5

TRANSMIT CONTROL
DATA TO
TRANSMITTING LINE   B6

STORABLE
REGION EXIST
?   B9

YES

NO

TRANSMIT IMAGE DATA
STORED IN IMAGE MEMORY
TO TRANSMITTING LINE — B10

F I G. 12